# FASCICULE DE BREVET EUROPEEN

(11) **EP 0 752 250 B1**
(45) Date de publication et mention de la délivrance du brevet: **03.09.2003**
(21) Numéro de dépôt: 96490030.2
(22) Date de dépôt: 05.07.1996
(51) Int. Cl.: A61L 11/00, A61L 2/18

(54) **Procédé de stérilisation des résidus corporels liquides**
Verfahren zur Sterilisierung von flüssigen Körperausscheidungen
Method of sterilising liquid body wastes

(30) Priorité: 06.07.1995 FR 9508470
(43) Date de publication de la demande: 08.01.1997
(73) Titulaire: C.M.E, SARL, 62410 Wingles (FR)
(72) Inventeur: Romelard, Daniel, 62410 Wingles (FR)
(74) Mandataire: Ecrepont, Robert

(56) Documents cités:
- EP-A- 0 586 763
- EP-A- 0 634 178
- FR-A- 2 670 676
- US-A- 5 078 965
- US-A- 5 087 420

## Description

L'invention se rapporte à un procédé de stérilisation des résidus corporels liquides.

Elle se rapporte également aux moyens en vue de la mise en oeuvre de ce procédé.

Les résidus corporels tels le sang, les urines, etc... que collectent les services hospitaliers, les laboratoires d'analyse ou encore les morgues lors des toilettages mortuaires ne sont pas toujours exempts de virus ou microbes qu'il faut nécessairement neutraliser avant de les rejeter notamment dans les égouts.

Pour cela, il est connu d'installer localement des dispositifs de collecte comprenant essentiellement un réservoir de collecte où les résidus sont momentanément accumulés.

Lorsque le réservoir est rempli, le contenu est traité dans un autre lieu où le contenu du réservoir est transvasé manuellement dans un système de traitement.

Cette opération présente des risques pour l'opérateur.

Pour tenter d'y remédier, on connaît un dispositif qui collecte et traite en même temps les résidus corporels (FR-A-2.670.676) et, à cet effet, avant chaque utilisation, on introduit dans le réservoir une quantité prédéterminée de désinfectant de sorte que les résidus sont introduits dans le désinfectant et sont traités par contact avec ledit désinfectant.

Selon ce procédé, les matières introduites en fin de remplissage du réservoir sont nécessairement moins longtemps en contact avec le désinfectant.

On sait par ailleurs que l'action des désinfectants dépend de la température et que plus la température est basse, plus longtemps les résidus doivent rester en contact avec le désinfectant.

Egalement, les produits introduits en dernier sont en contact moins longtemps avec le désinfectant que ceux introduits en premier lieu.

L'ajout progressif des matières fait dériver progressivement la température du désinfectant.

Pour obvier à cette différence le temps de mise en présence avec le produit de traitement et de température de traitement, la quantité de désinfectant introduite à chaque cycle est largement surévaluée pour garantir que toutes les matières y compris celles introduites en dernier bénéficieront d'un traitement minimal.

Le coût des produits désinfectant étant élevé, cela se répercute sur le coût d'utilisation de ces dispositifs.

On connaît un dispositif de traitement EP-A-0.634.178 comprenant deux chambres de traitement reliées par un syphon.

Ce dispositif comprend des moyens pour couper l'entrée des résidus dans la première chambre et pour transférer son contenu dans la seconde chambre.

Lorsque le contenu de la première chambre est transféré, un liquide désinfectant est admis dans la première chambre pour la nettoyer.

Lorsque la seconde chambre est vidée de la même manière que précédemment, une arrivée de désinfectant nettoye cette deuxième chambre.

Il n'existe pas réellement de traitement du liquide contenu dans ces différentes chambres mais une simple arrivée de désinfectant dans les chambres préalablement vidées.

Par US-A-5,087,420 et US-A-5,078,965 on connaît des dispositifs de traitement des liquides où la quantité de liquide à désinfecter est mesurée par une détection de niveau. Dans ces dispositifs, seulement lorsque le niveau de liquide atteint un niveau prédéfini le désinfectant est introduit et le traitement peut commencer.

Un des résultats que l'invention vise à obtenir est un procédé du type précité qui remédie aux inconvénients précités.

A cet effet, l'invention a pour objet un tel procédé de stérilisation des résidus humains liquides, selon lequel procédé on met en contact les résidus avec un produit désinfectant, ce procédé mettant en oeuvre :
- un réservoir de collecte d'une contenance prédéterminée,
- une conduite d'arrivée des résidus dans le réservoir,
- une conduite d'évacuation des résidus traités et sur laquelle est prévue une vanne de fermeture et,
- une conduite permettant d'introduire une quantité prédéterminée de désinfectant dans le réservoir,
selon lequel procédé :
- on met classiquement les résidus ainsi introduits en contact avec une quantité de désinfectant,
- pendant la durée du traitement, on interdit toute introduction de résidus supplémentaires dans le réservoir,
- également pendant toute la durée du traitement, on verrouille la vanne de fermeture placée sur la conduite d'évacuation, et
- à la fin du traitement, on déverrouille au moins la vanne de fermeture placée sur la conduite d'évacuation,
ce procédé étant caractérisé en ce que :
- on détermine la quantité de résidus dans le réservoir par une opération de pesée,
- on calcule la quantité de désinfectant correspondant à la quantité mesurée de résidus pour un traitement d'une durée prédéterminée,
- on introduit la quantité de désinfectant dans le réservoir, et
- on décompte le temps imparti au traitement pour, à la fin, évacuer les résidus désinfectés.

L'invention se rapporte également aux moyens en vue de la mise en oeuvre de ce procédé.

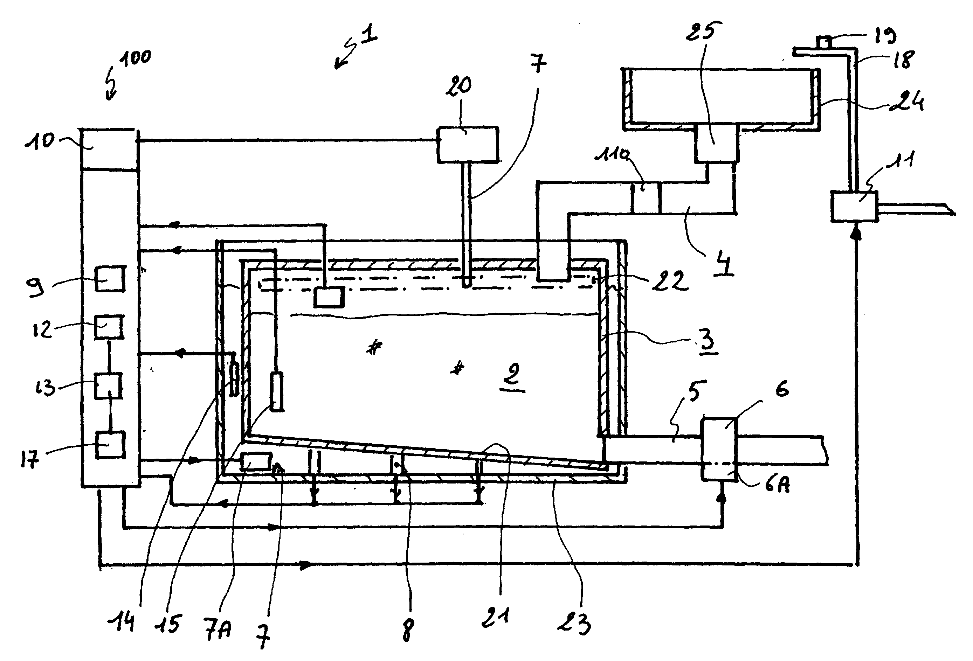

L'invention sera bien comprise à l'aide de la description ci-après faite à titre d'exemple non limitatif en regard du dessin ci-annexé qui représente schématiquement une installation de traitement des résidus corporels liquides.

En se reportant au dessin, on voit que le dispositif 1 de traitement par stérilisation des résidus corporels liquides 2 comprend :
- un réservoir de collecte 3 d'une contenance prédéterminée,
- une conduite 4 d'arrivée des résidus dans le réservoir 3,
- une conduite d'évacuation 5 des résidus traités sur laquelle est prévue une vanne 6 de fermeture et,
- une conduite 7 permettant d'introduire une quantité prédéterminée de désinfectant dans le réservoir 3.

Selon lequel procédé :
- on met les résidus ainsi introduits en contact avec une quantité de désinfectant,
- pendant la durée du traitement, on interdit toute introduction de résidus supplémentaires dans le réservoir 3,
- également pendant toute la durée du traitement, on verrouille la vanne 6 de fermeture placée sur la conduite d'évacuation 5, et
- à la fin du traitement, on déverrouille au moins la vanne 6 de fermeture placée sur la conduite 5 d'évacuation.

Selon une caractéristique essentielle de l'invention du procédé de l'invention :
- on détermine la quantité de résidus dans le réservoir par une opération de pesée,
- on calcule la quantité de désinfectant correspondant à la quantité de résidus mesurée pour un traitement d'une durée prédéterminée,
- on introduit la quantité de désinfectant dans le réservoir, et
- on décompte le temps imparti au traitement pour, à la fin, évacuer les résidus désinfectés.

De préférence :
- on amène au préalable la température des résidus à une valeur prédéterminée et lorsque cette température est atteinte, on commence à décompter le temps imparti au traitement,
- on trempe le réservoir dans un liquide par lequel, à la manière d'un bain marie, on chauffe le réservoir, et
- on surveille la température du réservoir et on adapte la température du "bain marie" selon l'évolution de cette température dans le réservoir 3.

Lorsque pour une raison ou une autre, le décompte du temps est interrompu avant la fin du traitement, on recommence le cycle de traitement depuis le début.

Lorsque, dans le réservoir a déjà été introduite une quantité de résidus comprise entre une valeur prédéterminée inférieure à la quantité maximale admissible et la quantité maximale admissible, on signale l'événement.

Par exemple, dans le cas d'un système associé à une table de toilettage mortuaire, cet avertissement sera fait dès que le volume restant à remplir sera de l'ordre du volume de liquide habituellement utilisé pour chaque toilettage.

Cela évite qu'au cours du toilettage, il soit nécessaire d'arrêter celui-ci pour cause de trop plein de la cuve.

Dans une forme de réalisation (non représentée), en amont du réservoir de collecte, on constitue des réservoirs tampons permettant de stocker provisoirement les résidus pendant que s'effectue le traitement dans le réservoir principal.

Après vidange du réservoir, on rince celui-ci avec une solution adaptée et ensuite on introduit dans le réservoir une faible quantité d'eau et de désinfectant.

Les moyens pour la mise en oeuvre du procédé comprennent notamment :
- des moyens 7 en vue d'amener et maintenir les résidus contenus dans ledit réservoir à une température prédéterminée,
- des moyens 8 pour peser la quantité de résidus contenus dans le réservoir avant d'entreprendre le traitement,
- des moyens 9 de détermination de la quantité de désinfectant adaptée à la quantité de résidus contenus dans le réservoir,
- des moyens 10 pour activer l'opération de stérilisation en introduisant la quantité calculée de désinfectant,
- des moyens 11,110 pour empêcher toute entrée de résidus pendant la durée du traitement,
- des moyens 6A pour interdire la manoeuvre de la vanne de fermeture placée sur la conduite 5 d'évacuation avant la fin du traitement.

Ils comprennent également des moyens 12 pour fixer un temps de traitement et des moyens 13 pour décompter le temps dès que le traitement a débuté.

De préférence, les moyens 7 pour chauffer les résidus dans le réservoir consistent en un bain de chauffage à la manière d'un "bain marie", ce qui évite les chocs thermiques sur les matières en contact avec l'élément chauffant 7A ainsi que cela serait à craindre si celui-ci était plongé directement dans le réservoir.

Les moyens comprennent également un moyen de mesure de la température dans le réservoir et un moyen de mesure de la température dans le bain, par exemple à l'aide de sondes 14, 15.

Les conduites d'entrée et d'évacuation sont pourvues d'électrovannes commandées par un microprocesseur 100 qui gère automatiquement le cycle de traitement.

Les moyens comprennent en outre des moyens 17 pour recommencer le cycle de traitement lorsque le traitement a été interrompu avant son terme, par exemple lors d'une coupure de courant.

Le dispositif peut équiper des hôpitaux mais également peut être incorporé à des tables destinées aux toilettes mortuaires.

Ces tables sont alors équipées de robinets 18 d'eau chaude et d'eau froide qui sont protégés par des dispositifs de sécurité (non représentés) tels des disconnecteurs avec des clapets antiretour évitant la pollution accidentelle du réseau de distribution.

Les robinets seront à commande non manuelle telle à commande 19 infra-rouge ou au pied.

Les conduites d'alimentation de ces robinets seront également pourvues d'électrovannes 11 qui couperont l'eau dès que le cycle de traitement est en cours.

Est également prévue une réserve 20 de désinfectant comprenant un dispositif d'alarme se déclenchant lorsque le contenu de cette réserve est inférieur à la quantité de désinfectant nécessaire.

Le fond du réservoir présente une pente 21 conformée pour que le point le plus bas se situe au niveau de son raccordement avec la conduite d'évacuation.

Une rampe 22 de rinçage située en partie supérieure du réservoir permet de rincer ledit réservoir après vidange.

La cuve 23 contenant le bain marie constituera une deuxième enveloppe garantissant l'étanchéité du système.

En amont de la conduite d'entrée des résidus dans le réservoir et en aval d'un bac 24 de réception où l'opérateur déverse les résidus est, éventuellement, prévu un broyeur 25.

Ce procédé et ces moyens de mise en oeuvre garantissent que les résidus seront traités suffisamment avant d'être rejetés tout en ne consommant qu'une quantité de désinfectant adaptée.

Tout contact avec les résidus non-stérilisés disposés dans le réservoir est rendu impossible et toute élimination de ces résidus sans traitement est également rendue impossible.

## Revendications

1. Procédé de stérilisation des résidus corporels liquides mettant en oeuvre :
- un réservoir de collecte (3),
- une conduite (4) d'arrivée des résidus dans le réservoir (3),
- une conduite d'évacuation (5) des résidus traités sur laquelle est prévue une vanne (6) de fermeture et,
- une conduite (7) permettant d'introduire une quantité de désinfectant dans le réservoir (3),
selon lequel procédé :
- pendant la durée du traitement, les résidus sont en contact avec le désinfectant et on interdit toute introduction de résidus supplémentaires dans le réservoir (3), et,
- on verrouille la vanne (6) de fermeture placée sur la conduite d'évacuation (5),
ce procédé étant **CARACTERISE en ce que** :
- on mesure la quantité de résidus contenue dans le réservoir par une opération de pesée,
- on calcule la quantité de désinfectant correspondant à la quantité de résidus mesurée pour la durée du traitement choisie,
- on introduit alors la quantité de désinfectant dans le réservoir, et
- on décompte le temps imparti au traitement pour, à la fin, évacuer les résidus désinfectés.

2. Procédé selon la revendication 1 **caractérisé en ce qu'**on trempe le réservoir dans un liquide par lequel, à la manière d'un bain marie, on chauffe le réservoir pour amener les résidus à la température de traitement et lorsque la température de traitement est atteinte, on commence à décompter le temps imparti au traitement et, d'autre part,
- on surveille la température du réservoir et on adapte la température du "bain marie" selon l'évolution de cette température dans le réservoir (3).

3. Procédé selon la revendication 1 ou 2 **caractérisé en ce que**, lorsque pour une raison ou pour une autre, le décompte du temps est interrompu avant la fin du traitement, on recommence le cycle de traitement depuis le début.

4. Procédé selon la revendication 1 **caractérisé en ce que**, lorsque, dans le réservoir a déjà été introduire une quantité de résidus comprise entre une valeur prédéterminée inférieure à la quantité maximale admissible et la quantité maximale admissible, on signale l'évènement.

5. Procédé selon la revendication 1 **caractérisé en ce que**, en amont du réservoir de collecte, on constitue des réservoirs tampons permettant de stocker provisoirement les résidus pendant que s'effectue le traitement dans le réservoir principal.

6. Procédé selon la revendication 1 **caractérisé en ce que**, après vidange du réservoir, on rince celui-ci avec une solution adaptée et on introduit dans le réservoir une faible quantité d'eau et de désinfectant.

7. Moyens pour la mise en oeuvre du procédé selon la revendication 1 comprenant:
- un réservoir de collecte (3) d'une contenance prédéterminée,
- une conduite (4) d'arrivée des résidus dans le réservoir (3),
- une conduite d'évacuation (5) des résidus traités sur laquelle est prévue une vanne (6) de fermeture et,
- une conduite (7) permettant d'introduire une quantité prédéterminée de désinfectant dans le réservoir (3),
- des moyens (11, 110) pour empêcher toute entrée de résidus pendant la durée du traitement, et
- des moyens (6A) pour interdire la manoeuvre de la vanne de fermeture placée sur la conduite (5) d'évacuation avant la fin du traitement,
ces moyens étant **caractérisés en ce qu'**ils comprennent :
- des moyens (8) pour peser la quantité de résidus contenus dans le réservoir avant d'entreprendre le traitement,
- des moyens (9) de détermination de la quantité de désinfectant adaptée à la quantité de résidus contenus dans le réservoir, et
- des moyens (10) pour activer l'opération de stérilisation en introduisant la quantité calculée de désinfectant.

8. Moyens selon la revendication 7 **caractérisés en ce qu'**ils comprennent des moyens (7) en vue d'amener et maintenir les résidus contenus dans ledit réservoir à une température prédéterminée.

9. Moyens selon la revendication 8 **caractérisés en ce que** les moyens (7) pour chauffer les résidus dans le réservoir consistent en un bain de chauffage à la manière d'un "bain marie".

10. Moyens selon l'une quelconque des revendications 7 à 9 **caractérisés en ce qu'**ils comprennent des moyens (12) pour fixer un temps de traitement et des moyens (13) pour décompter le temps dès que le traitement a débuté.

## Patentansprüche

1. Verfahren zum Sterilisieren von Rückständen von Körperflüssigkeiten, bei welchem folgende Einrichtungen zum Einsatz kommen:
- ein Auffangbehälter (3),
- eine Zuführleitung (4) zum Zuführen der Rückstände in den Behälter (3),
- eine Auslassleitung (5) zum Auslassen der behandelten Rückstände, auf welcher ein Ventilschieber (6) zum Verschließen vorgesehen ist, und
- eine Leitung (7), welche die Einleitung einer Menge an Desinfektionsmittel in den Behälter (3) ermöglicht,
wobei bei diesem Verfahren:
- während der Dauer der Behandlung die Rückstände mit dem Desinfektionsmittel in Kontakt stehen und jegliche Einleitung zusätzlicher Rückstände in den Behälter (3) unmöglich ist, und
- der Ventilschieber (6) zum Verschließen, der auf der Auslassleitung (5) angeordnet ist, verriegelt wird,
**dadurch GEKENNZEICHNET, dass**:
- die Menge der im Behälter aufgenommenen Rückstände in einem Wiegevorgang erfasst wird,
- die Menge des Desinfektionsmittels, die der erfassten Menge an Rückständen entspricht, für die gewählte Dauer der Behandlung berechnet wird,
- nun die Menge an Desinfektionsmittel in den Behälter eingeleitet wird, und
- der Ablauf der für die Behandlung zugewiesenen Zeit über einen Zähler erfasst wird, um schließlich die desinfizierten Rückstände abzulassen.

2. Verfahren nach Anspruch 1, **dadurch gekennzeichnet, dass**
- der Behälter in eine Flüssigkeit eingetaucht wird, mit deren Hilfe der Behälter in der Art eines Wasserbades erwärmt wird, um so die Rückstände auf die Behandlungstemperatur zu bringen, und dass dann, wenn die Behandlungstemperatur erreicht ist, die Erfassung des Ablaufs der für die Behandlung zugewiesenen Zeit beginnt, und dass zum anderen
- die Temperatur des Behälters überwacht wird und die Temperatur des "Wasserbades" entsprechend der Entwicklung dieser Temperatur im Behälter (3) angepasst wird.

3. Verfahren nach Anspruch 1 oder 2, **dadurch gekennzeichnet, dass** dann, wenn aus irgendeinem Grund die Erfassung der Zeit vor Beendigung der Behandlung unterbrochen wird, der Behandlungszyklus von Anfang an wieder aufgenommen wird.

4. Verfahren nach Anspruch 1, **dadurch gekennzeichnet, dass** dann, wenn in den Behälter bereits eine Menge an Rückständen eingeleitet wurde, die zwischen einem vorgegebenen unteren Wert, der kleiner als die höchstzulässige Menge ist, und dem höchstzulässigen Wert liegt, eine Meldung dieses Ereignisses erfolgt.

5. Verfahren nach Anspruch 1, **dadurch gekennzeichnet, dass** auf der Zuleitungsseite des Auffangbehälters Pufferbehälter gebildet werden, welche die vorübergehende Zwischenspeicherung der Rückstände ermöglichen, während die Behandlung im Hauptbehälter abläuft.

6. Verfahren nach Anspruch 1, **dadurch gekennzeichnet, dass** nach der Entleerung des Behälters dieser mit einer geeigneten Lösung ausgespült wird und in den Behälter eine geringe Menge an Wasser und Desinfektionsmittel eingeleitet wird.

7. Einrichtungen zur Durchführung des Verfahrens nach Anspruch 1, welche folgendes umfassen:
- ein Auffangbehälter (3) mit vorgegebenem Fassungsvermögen,
- eine Zuführleitung (4) zum Zuführen der Rückstände in den Behälter (3),
- eine Auslassleitung (5) zum Auslassen der behandelten Rückstände, auf welcher ein Ventilschieber (6) zum Verschließen vorgesehen ist, und
- eine Leitung (7), welche die Einleitung einer Menge an Desinfektionsmittel in den Behälter (3) ermöglicht,
- sowie Einrichtungen (11, 110), um jegliches Eindringen von Rückständen während der Dauer der Behandlung zu verhindern, und
- Einrichtungen (6A), um eine Betätigung des Ventilschiebers zum Verschließen, der auf der Auslassleitung (5) angeordnet ist, vor Beendigung der Behandlung unmöglich zu machen,
**dadurch gekennzeichnet, dass** die Einrichtungen folgendes aufweisen:
- Mittel (8) zum Erfassen der Menge der im Behälter aufgenommenen Rückstände vor Aufnahme der Behandlung,
- Mittel (9) zum Bestimmen der Menge des Desinfektionsmittels, die an die erfasste Menge an Rückständen angepasst ist, welche im Behälter aufgenommen sind, und
- Mittel (10) zum Aktivieren des Sterilisiervorgangs unter Einleitung der berechneten Menge an Desinfektionsmittel.

8. Einrichtungen nach Anspruch 7, **dadurch gekennzeichnet, dass** sie Mittel (7) umfassen, um die in dem Behälter aufgenommenen Rückstände auf eine vorgegebene Temperatur zu bringen und auf diesem Temperaturwert zu halten.

9. Einrichtungen nach Anspruch 8, **dadurch gekennzeichnet, dass** die Mittel (7) zum Erwärmen der Rückstände in dem Behälter aus einem Erwärmungsbad in der Art eines "Wasserbades" bestehen.

10. Einrichtungen nach einem der Ansprüche 7 bis 9, **dadurch gekennzeichnet, dass** sie Mittel (12) umfassen, um eine Behandlungszeit festzulegen, sowie Mittel (13), um die ab Beginn der Behandlung abgelaufene Zeit zu erfassen.

## Claims

1. A method of sterilising liquid body waste, using:
- a collection container (3),
- a supply line (4) for supplying waste to the container (3),
- a drainage line (5) for draining off treated waste, in which a closing valve (6) is provided, and
- a line (7) allowing a quantity of disinfectant to be introduced into the container (3),
according to which method:
- during the course of treatment, the waste is in contact with the disinfectant and any introduction of additional waste into the container (3) is prevented, and
- the closing valve (6) positioned in the drainage line (5) is blocked,
this method being **characterised in that**
- the quantity of waste in the container is measured in a weighing operation,
- the quantity of disinfectant corresponding to the quantity of waste measured is calculated for the selected duration of the treatment,
- the quantity of disinfectant is then introduced into the container, and
- the time allowed for treatment is counted down and finally the disinfected waste is drained off.

2. A method according to Claim 1, **characterised in that** the container is dipped into a liquid which is used to heat the container in the manner of a bain marie, in order to bring the waste to the treatment temperature, and once the treatment temperature has been reached the count-down of the time allowed for treatment begins, and on the other hand
- the temperature of the container is monitored and the temperature of the "bain marie" is adapted as a function of a change to this temperature in the container (3).

3. A method according to Claim 1 or 2, **characterised in that**, in the event for one reason or another that the count-down of time is interrupted before the end of treatment, the treatment cycle is restarted from the beginning.

4. A method according to Claim 1, **characterised in that**, in the event that a quantity of waste between a predetermined value below the maximum permissible quantity and the maximum permissible quantity has already been introduced into the container, this is signalled.

5. A method according to Claim 1, **characterised in that**, upstream of the collection container, buffer containers are formed, allowing the waste to be stored temporarily while the treatment is being carried out in the main container.

6. A method according to Claim 1, **characterised in that** once the container has been emptied it is rinsed with a suitable solution and a small quantity of water and disinfectant is introduced into the container.

7. Means of implementing the method according to Claim 1, comprising:
- a collection container (3) of predetermined capacity,
- a supply line (4) for supplying waste to the container (3),
- a drainage line (5) for draining off treated waste, in which a closing valve (6) is provided,
- a line (7) allowing a predetermined quantity of disinfectant to be introduced into the container (3),
- means (11, 110) for preventing any waste from entering during the course of treatment, and
- means (6A) for preventing the closing valve positioned in the drainage line (5) from being actuated before the end of treatment,
these means being **characterised in that** they comprise:
- means (8) for weighing the quantity of waste in the container before treatment is undertaken,
- means (9) for determining the quantity of disinfectant adapted to the quantity of waste in the container, and
- means (10) for activating the sterilisation operation by introducing the calculated quantity of disinfectant.

8. Means according to Claim 7, **characterised in that** they comprise means (7) for the purpose of bringing the waste in the said container to a predetermined temperature and keeping it at that temperature.

9. Means according to Claim 8, **characterised in that** the means (7) for heating the waste in the container are composed of a heating bath in the manner of a "bain marie".

10. Means according to any one of Claims 7 to 9, **characterised in that** they comprise means (12) for setting a treatment time and means (13) for counting down the time once treatment has begun.
